(19) Europäisches Patentamt
European Patent Office
Office européen des brevets

(11) **EP 4 643 812 A1**

(12) **EUROPEAN PATENT APPLICATION**
published in accordance with Art. 153(4) EPC

(43) Date of publication:
**05.11.2025 Bulletin 2025/45**

(21) Application number: **23912907.5**

(22) Date of filing: **27.12.2023**

(51) International Patent Classification (IPC):
*A61C 8/00* (2006.01)      *A61L 27/32* (2006.01)
*A61L 27/06* (2006.01)      *A61L 27/30* (2006.01)
*A01N 59/06* (2006.01)      *A01N 59/16* (2006.01)
*A01N 59/26* (2006.01)      *A01N 59/08* (2006.01)

(52) Cooperative Patent Classification (CPC):
A01N 59/06; A01N 59/08; A01N 59/16;
A01N 59/26; A61C 8/00; A61L 27/06; A61L 27/30;
A61L 27/32

(86) International application number:
**PCT/KR2023/021640**

(87) International publication number:
**WO 2024/144233 (04.07.2024 Gazette 2024/27)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **30.12.2022 KR 20220190477
30.12.2022 KR 20220190625**

(71) Applicant: **Osstemimplant Co., Ltd.
Gangseo-gu
Seoul 07789 (KR)**

(72) Inventors:
• **HA, Kyung Won
Seoul 07789 (KR)**
• **HAN, Jae Won
Seoul 07789 (KR)**

(74) Representative: **Germain Maureau
12, rue Boileau
69006 Lyon (FR)**

(54) **DENTAL IMPLANT WITH EXCELLENT OSSEOINTEGRATION AND ANTIBACTERIAL PROPERTIES AND ANTIBACTERIAL COATING METHOD FOR TITANIUM SUBSTRATE**

(57)      Disclosed are a dental implant and an antibacterial coating method for a titanium substrate, the dental implant comprising: a titanium substrate; and a coating layer formed on at least a portion of the surface of the titanium substrate and comprising a silver-apatite complex, wherein the Ag/Ca molar ratio of the coating layer is 0.3 to 0.6, and the Ca/P molar ratio is 0.9 to 1.1.

[Fig. 1]

**Description**

[Technical Field]

The present invention relates to a dental implant with excellent osseointegration and antibacterial properties, and an antibacterial coating method for a titanium substrate.

[Background Art]

A dental implant is an artificial tooth that can permanently replace a missing tooth and is widely used to restore the masticatory function in partially or fully edentulous areas.

Most implants are made of either ceramic or metal materials, and among them, titanium materials are primarily used due to its physical properties being similar to human bone and its excellent resistance to corrosion. However, titanium materials have issues such as lower biocompatibility compared to ceramic materials.

Recently, various methods have been developed to obtain dental implants with excellent mechanical strength and biocompatibility by coating the surface of titanium materials with bioactive materials.

Apatite, which is mainly used as a bioactive material, has excellent biocompatibility because it is identical to the main component of bone and teeth, and it exhibits excellent osseointegration, allowing stable bonding with bone after implantation.

Methods for coating titanium materials with apatite include plasma spraying, sputtering, ion implantation, and ion beam deposition. However, these coating methods have the problem that it is difficult to uniformly deposit a coating layer. Therefore, further research is needed on coating methods that can maintain surface morphology for a long period in the human body and are suitable for bone formation and osseointegration.

Meanwhile, since the fixation force of a dental implant is determined by the quantity and quality of the patient's bone, it is important to enhance stability after implant placement.

The stability of an implant is expressed as the sum of primary stability, which occurs when the implant comes into contact with the surrounding bone, and secondary stability, which is obtained due to the formation of new bone tissue and osseointegration after implant placement. In particular, increasing the early stability after implant placement is a key factor in enhancing mid- to long-term stability and, ultimately, the success rate of the implant.

The main causes of early implant failure include various factors such as product defects, surgical factors, and patient-related factors, as well as loss of bone surrounding the dental implant or a decreased fixation force. In particular, when the bone surrounding the implant is lost, the fixation force is inevitably decreased. As a result of analyzing early failure cases due to bone loss surrounding the implant, it was found that about 90% of early failures were due to periodontitis or bacterial infection, and analysis of products retrieved from customers showed that anaerobic bacteria were detected in about 60% of the cases.

To solve the above problems, it is necessary to impart antibacterial properties to the implant surface for active defense against contaminated saliva and bacteria, and to maintain antibacterial properties even during the initial implantation period.

However, there are technical limitations in that antibacterial substances applied to the implant surface may reduce the bone-forming ability of the implant.

Therefore, there is a need to develop dental implants with excellent osseointegration and antibacterial properties, as well as antibacterial coating methods for titanium substrates.

[Disclosure]

[Technical Problem]

The present invention is intended to solve the above-described problems, and its object is to provide a dental implant with excellent osseointegration and antibacterial properties, and an antibacterial coating method for a titanium substrate.

[Technical Solution]

One aspect of the present invention provides a dental implant including: a titanium substrate; and a coating layer formed on at least a part of a surface of the titanium substrate and including a silver-apatite composite, wherein the coating layer has an Ag/Ca molar ratio of 0.3 to 0.6 and a Ca/P molar ratio of 0.9 to 1.1.

In an embodiment, the coating layer may be composed of apatite and a silver-apatite composite.

In an embodiment, the coating layer may not include metallic silver.

In an embodiment, the coating layer may have an average thickness of 50 nm or less.

Another aspect of the present invention provides an antibacterial coating method for a titanium substrate, including: (a) preparing a solution including a calcium precursor, a silver precursor, and a phosphate precursor; and (b) immersing a titanium substrate in the solution to form a coating layer including a silver-apatite composite on the surface of the titanium substrate, wherein the solution has an Ag/Ca molar ratio of 0.3 to 0.6 and a Ca/P molar ratio of 0.9 to 1.1.

In an embodiment, the calcium precursor may be one or more selected from the group consisting of calcium nitrate ($Ca(NO_3)_2$), calcium carbonate ($CaCO_3$), calcium hydroxide ($Ca(OH)_2$), calcium oxide ($CaO$), and calcium chloride ($CaCl_2$).

In an embodiment, the silver precursor may be one or more selected from the group consisting of silver nitrate ($AgNO_3$), silver perchlorate ($AgClO_4$), silver tetrafluoroborate ($AgBF_4$), silver hexafluorophosphate ($AgPF_6$), silver acetate ($CH_3COOAg$), silver trifluoromethanesulfonate ($AgCF_3SO_3$), silver sulfate ($Ag_2SO_4$), silver 2,4-pentanedionate ($CH_3COCH=COCH_3Ag$), and silver oxides ($AgO$, $Ag_2O$, $Ag_2O_2$, and $Ag_2O_3$).

In an embodiment, the phosphate precursor may be one or more selected from the group consisting of diammonium phosphate, disodium phosphate, and dipotassium phosphate.

In an embodiment, before step (b), the antibacterial coating method may further include pretreating the titanium substrate by one or more methods selected from the group consisting of sandblasting, anodizing, acid or base treatment, heat treatment, UV treatment, and plasma treatment.

In an embodiment, step (b) may be performed at a temperature of 10 to 40 °C for 40 to 180 minutes.

In an embodiment, after step (b), the antibacterial coating method may further include post-treating the titanium substrate on which the coating layer is formed by one or more methods selected from the group consisting of immersion in ethanol and ultrasonic cleaning.

[Advantageous Effects]

A dental implant according to the present invention exhibits excellent osseointegration and antibacterial properties, which can reduce the early failure rate of the implant, prevent delamination of the implant coating layer, and consequently increase the success rate of the implant.

The effects described in this specification are not limited to those mentioned above, and should be understood to include all effects that can be inferred from the configuration described in the detailed description or the claims of this specification.

[Description of Drawings]

FIG. 1 is a graph evaluating antibacterial performance for selecting an Ag-apatite solution according to an embodiment of the present invention.

FIG. 2 is a graph evaluating the residual amount of apatite after antibacterial performance evaluation in the example and comparative example 2.

FIG. 3 shows FE-SEM analysis images of the example and comparative example.

FIG. 4 shows EDS analysis images of the example and comparative example.

FIG. 5 is an XPS analysis graph of the example and the comparative example.

FIG. 6 is a graph evaluating antibacterial performance for selecting antibacterial ions in this specification.

FIG. 7 is a graph showing removal torque for selecting a method of forming a silver-titanium oxide matrix.

FIG. 8 is a graph evaluating antibacterial performance according to hydrothermal treatment conditions.

FIG. 9 shows FE-SEM analysis images of a titanium substrate with a silver-titanium oxide matrix.

FIG. 10 presents the elemental distribution of titanium and silver by location in the SEM image of FIG. 9.

FIG. 11 shows the EDS analysis results of a titanium substrate with a silver-titanium oxide matrix.

FIG. 12 is an XPS analysis graph of a titanium substrate with a silver-titanium oxide matrix.

FIG. 13A shows FE-SEM images of Example 4, FIG. 13B shows FE-SEM images of Comparative Example 4, and FIG. 13C shows FE-SEM images of Comparative Example 5.

FIG. 14 is a graph showing the results of measuring the amount of Ag released over 90 days for the specimens of Preparation Example 1, Example 1, and Example 4.

FIG. 15 is a graph showing the results of measuring the inhibition rate of salivary bacterial contamination for the specimens of Preparation Example 1, Example 1, and Example 4.

[Modes of the Invention]

Hereinafter, one aspect of this specification will be described with reference to the attached drawings. However, the content of this specification may be embodied in various different forms and are therefore not limited to the embodiments described herein. In addition, for clarity of explanation regarding one aspect of this specification in the drawings, parts unrelated to the description are omitted, and similar parts are given similar reference numerals throughout the specification.

In this specification, when a part is said to "include" a certain component, this does not mean that other components are excluded, but it means that other components can be added, unless specifically stated to the contrary.

When a numerical value range is described herein, unless a specific range is otherwise defined, the value is considered to have the precision indicated by the number of significant figures, in accordance with standard rules for significant digits in chemistry. For example, the value 10 represents a range from 5.0 to 14.9, and the value 10.0 represents a range from 9.50 to 10.49.

<First embodiment>

Hereinafter, a dental implant according to one aspect of the present specification will be described in detail.

One aspect of the present invention provides a dental implant including a titanium substrate and a coating layer formed on at least a part of a surface of the titanium substrate and including a silver-apatite composite, wherein the coating layer has an Ag/Ca molar ratio of 0.3 to 0.6 and a Ca/P molar ratio of 0.9 to 1.1.

The dental implant of the present invention exhibits excellent osseointegration and antibacterial properties by including a coating layer containing a silver-apatite composite, thereby reducing the early failure rate of the implant, inhibiting delamination of the implant coating layer, and consequently, increasing the success rate of the implant. Additionally, the coating layer may be naturally resorbed by osteoclasts during the bone regeneration process after implantation, thereby enabling stable seating of the implant.

Titanium substrate

The titanium substrate refers to a material composed of pure titanium or an alloy of titanium and other metals from the periodic table. The titanium alloy may be, for example, an alloy of titanium (Ti) and one element selected from the group consisting of aluminum (Al), silicon (Si), vanadium (V), niobium (Nb), zirconium (Zr), molybdenum (Mo), chromium (Cr), tin (Sn), tantalum (Ta), palladium (Pd), and combinations thereof, but is not limited thereto.

Coating layer including silver-apatite composite

The dental implant of the present invention exhibits excellent osseointegration and antibacterial properties by including a coating layer containing a silver-apatite composite, thereby reducing the early failure rate of the implant, inhibiting delamination of the implant coating layer, and consequently, increasing the success rate of the implant. Additionally, the coating layer may be naturally resorbed by osteoclasts during the bone regeneration process after implantation, thereby enabling stable seating of the implant.

The coating layer may have an Ag/Ca molar ratio of 0.3 to 0.6, preferably 0.35 to 0.55, and more preferably 0.4 to 0.5, but it is not limited thereto. When the Ag/Ca molar ratio is within this range, the rate of forming apatite crystals during the formation of the coating layer slows down, providing a favorable condition for the generation of silver-apatite, and as a result, excellent antibacterial properties may be achieved.

The coating layer may have a Ca/P molar ratio of 0.9 to 1.1, preferably 0.92 to 1.08, more preferably 0.95 to 1.05, and most preferably 0.98 to 1.02, but it is not limited thereto. When the Ca/P molar ratio is within this range, the deviation in osseointegration is reduced, thereby making it easy to control the quality of implant products.

The coating layer may not contain metallic silver and may be composed of apatite and a silver-apatite composite. In this case, the apatite may be low-crystalline apatite. When the coating layer consists of low-crystalline apatite and a silver-apatite composite, it does not dissolve in environments in which calcium and phosphorus ions are present, such as in the body, and may be completely resorbed by osteoclasts (pH 4) during the bone regeneration process. As a result, newly formed bone attaches directly to the surface of the implant rather than to the coating layer, thereby preventing implant failure due to bacterial infection during the early stage of implantation, maintaining the osteoconductivity of the low-crystalline apatite, and preventing implant loss due to delamination of the coating layer.

The coating layer may have an average thickness of 50 nm or less, preferably 40 nm or less, more preferably 30 nm or less, and most preferably 20 nm or less, but it is not limited thereto. When the average thickness of the coating layer is excessively large (for example, 100 nm or more), body fluids infiltrate micro-gaps caused by friction with bone during the implantation process, and these micro-gaps further widen during continuous mastication (chewing), which may cause implant failure due to delamination of the coating layer. On the other hand, when the average thickness of the coating layer is within the above range, implant failure due to delamination of the coating layer caused by friction with bone during implantation or by masticatory activity after implantation may be prevented.

Although the present invention does not particularly limit the method for forming the coating layer, for example, the coating layer may be formed using an antibacterial coating method that directly grows apatite on the titanium substrate. Hereinafter, this method will be described in detail below.

Antibacterial coating method for titanium substrate

Generally, well-known methods for coating titanium substrates with apatite include plasma spraying, sputtering, ion implantation, and ion beam deposition. However, each of these coating methods has limitations, such as difficulty in achieving uniform deposition of the coating layer and insufficient biocompatibility.

According to another aspect of the present invention, the antibacterial coating method for a titanium substrate involves direct growth of apatite on the titanium substrate to maintain osteoconductivity, thereby not only exhibiting excellent adhesion between the newly formed bone and the coating layer, but also preventing implant failure caused by bacteria during the early stage of implantation by incorporating silver ions to form a silver-apatite composite.

Specifically, the antibacterial coating method for a titanium substrate according to another aspect of the present invention includes: (a) preparing a solution including a calcium precursor, a silver precursor, and a phosphate precursor; and (b) immersing a titanium substrate in the solution to form a coating layer including a silver-apatite composite on the surface of the titanium substrate.

The calcium precursor included in the solution in step (a) may be, for example, one or more selected from the group consisting of calcium nitrate ($Ca(NO_3)_2$), calcium carbonate ($CaCO_3$), calcium hydroxide ($Ca(OH)_2$), calcium oxide (CaO), and calcium chloride ($CaCl_2$), and preferably calcium nitrate ($Ca(NO_3)_2$), but is not limited thereto.

The silver precursor included in the solution in step (a) may be, for example, one or more selected from the group consisting of silver nitrate ($AgNO_3$), silver perchlorate ($AgClO_4$), silver tetrafluoroborate ($AgBF_4$), silver hexafluoropho-sphate ($AgPF_6$), silver acetate ($CH_3COOAg$), silver trifluoromethanesulfonate ($AgCF_3SO_3$), silver sulfate ($Ag_2SO_4$), silver 2,4-pentanedionate ($CH_3COCH=COCH_3Ag$), and silver oxides (AgO, $Ag_2O$, $Ag_2O_2$, and $Ag_2O_3$), and preferably silver nitrate ($AgNO_3$), but is not limited thereto.

The phosphate precursor included in the solution in step (a) may be one or more selected from the group consisting of diammonium phosphate, disodium phosphate, and dipotassium phosphate, but is not limited thereto.

In step (a), the Ag/Ca molar ratio of the solution may be 0.3 to 0.6, and the Ca/P molar ratio of the solution may be 0.9 to 1.1. Since the ranges of the Ag/Ca and Ca/P molar ratios overlap those of the coating layer of the dental implant described above, a detailed description thereof will be omitted.

The temperature and time conditions of step (b) may affect the coating amount of the coating layer including the silver-apatite composite and, consequently, osseointegration.

Step (b) may be performed at a temperature of 10 to 40 °C. For example, it may be performed at 10 °C, 11 °C, 12 °C, 13 °C, 14 °C, 15 °C, 16 °C, 17 °C, 18 °C, 19 °C, 20 °C, 21 °C, 22 °C, 23 °C, 24 °C, 25 °C, 26 °C, 27 °C, 28 °C, 29 °C, 30 °C, 31 °C, 32 °C, 33 °C, 34 °C, 35 °C, 36 °C, 37 °C, 38 °C, 39 °C, 40 °C, or at a value between any two of these values, but is not limited thereto. When the temperature is too low, it may take a long time to form the coating layer. On the other hand, when the temperature is too high, delamination may occur, causing the coating layer with attached bone to separate from the implant surface.

Step (b) may be performed for 40 to 180 minutes. For example, it may be performed for 40 minutes, 41 minutes, 42 minutes, 43 minutes, 44 minutes, 45 minutes, 46 minutes, 47 minutes, 48 minutes, 49 minutes, 50 minutes, 51 minutes, 52 minutes, 53 minutes, 54 minutes, 55 minutes, 56 minutes, 57 minutes, 58 minutes, 59 minutes, 60 minutes, 61 minutes, 62 minutes, 63 minutes, 64 minutes, 65 minutes, 66 minutes, 67 minutes, 68 minutes, 69 minutes, 70 minutes, 71 minutes, 72 minutes, 73 minutes, 74 minutes, 75 minutes, 76 minutes, 77 minutes, 78 minutes, 79 minutes, 80 minutes, 81 minutes, 82 minutes, 83 minutes, 84 minutes, 85 minutes, 86 minutes, 87 minutes, 88 minutes, 89 minutes, 90 minutes, 91 minutes, 92 minutes, 93 minutes, 94 minutes, 95 minutes, 96 minutes, 97 minutes, 98 minutes, 99 minutes, 100 minutes, 110 minutes, 120 minutes, 130 minutes, 140 minutes, 150 minutes, 160 minutes, 170 minutes, 180 minutes, or for a value between any two of these values, but is not limited thereto. When the coating time is too short, the coating may not be applied properly to the implant or the adhesion of the coating layer may be reduced. On the other hand, when the coating time is too long, the coating layer may become excessively thick, which may cause implant failure due to delamination of the coating layer.

Before step (b), pretreating the titanium substrate by one or more methods selected from the group consisting of sandblasting, anodizing, acid or base treatment, heat treatment, UV treatment, and plasma treatment may be further included. In this case, such pretreatment may be advantageous for osseointegration and/or antibacterial properties, but is not limited thereto.

After step (b), post-treating the titanium substrate on which the coating layer is formed by one or more methods selected from the group consisting of ethanol immersion and ultrasonic cleaning may be further included. In this case, such post-treatment may be advantageous for osseointegration and/or antibacterial properties, but is not limited thereto.

<Second embodiment>

Hereinafter, a dental implant according to another aspect of the present specification will be described in detail. However, descriptions that overlap the above first embodiment will be omitted.

Another aspect of the present invention provides a dental implant including: a titanium substrate; a silver-titanium oxide matrix formed on at least a part of the surface of the titanium substrate; and a coating layer formed on at least a part of the surface of the silver-titanium oxide matrix and including a silver-apatite composite.

The dental implant may release silver at a rate of 1 ppm/day or less for at least one week in an inorganic solvent at 37 °C. The sustained release effect of the dental implant may last for at least one week, one month, two months, three months, or longer. The sustained release effect of the dental implant may refer to the long-term release of at least one of silver ions and metallic silver, thereby realizing a continuous antibacterial effect. The inorganic solvent may be water, such as purified water or ion-exchanged water. The dental implant may release silver at a rate of at least 1 ppb/day, 5 ppb/day, or 10 ppb/day during the specified period.

Silver-titanium oxide matrix

To increase the success rate of dental implant, it is necessary to establish stable integration with the surrounding bone of a user during the initial implantation period. Among the various factors of implantation failure, early failure due to bacterial infection accounts for a significant proportion.

The dental implant of the present invention includes a silver-titanium oxide matrix formed on at least a part of the titanium substrate. The silver-titanium oxide matrix exhibits a peak $P_1$ at a binding energy of 570 to 580 eV and a peak $P_2$ at a binding energy of 360 to 375 eV, as measured by XPS, and the intensities $I_1$ of $P_1$ and $I_2$ of $P_2$ satisfy the following Expression, thereby exhibiting excellent antibacterial properties.

$$[\text{Expression}]$$

$$2*I_1 \leq I_2 \leq 5*I_1$$

The silver-titanium oxide matrix may inhibit the formation of a biofilm on the surface of the implant by blocking quorum sensing. As a result, the early failure rate of the implant may be reduced, delamination of the implant coating layer may be prevented, and ultimately, the success rate of the implant may be improved.

The term "quorum sensing" refers to a bacterial density-dependent gene expression regulation mechanism in which the collective metabolic activity of bacteria is controlled when a certain concentration is reached, is proportional to cell density during bacterial proliferation and accumulates. In other words, when the cell density reaches a so-called quorum for decision-making through proliferation, specific traits that are not observed in low-density cells are collectively induced and expressed. In particular, pathogenic bacteria may cause infection by inducing virulence through quorum sensing.

The silver-titanium oxide matrix may kill bacteria by releasing silver ions, which are antibacterial ions. As a result, it is possible to inhibit the attachment of bacteria to the surface of the implant. The silver ions may bind to cysteine groups in the bacterial membrane and inactivate proteins. They may induce the release of reactive oxygen species from the bound cells, thereby killing the bacteria. Through the above-described mechanisms, silver ions may effectively suppress biofilm formation by killing bacteria and may continuously maintain antibacterial properties without inducing antibacterial resistance.

The silver-titanium oxide matrix may include antibacterial components in various forms. As a result, it may provide the level of antibacterial properties required in the early stage of implantation and may maintain long-term antibacterial effects.

The peak $P_1$ and intensity $I_1$ at a binding energy of 570 to 580 eV may indicate the range and intensity values of the Ag 2p peak. The peak $P_2$ and intensity $I_2$ at 360 to 375 eV may indicate the range and intensity values of the Ag 3d peak. The Ag 2p peak may represent metallic silver, and the Ag 3d peak may represent oxidized silver ions. A silver-titanium oxide matrix, whose intensities $I_1$ and $I_2$ at these peaks satisfy specific conditions may simultaneously contain metallic silver and silver ions, thereby achieving a sustained release effect. In addition, when $I_1$ and $I_2$ satisfy specific conditions, the early success rate of the dental implant placement may be effectively increased.

The conditions of $I_1$ and $I_2$, as expressed in the aforementioned Expression, may refer to the ratio between metallic silver and silver ions measured on the XPS graph. The value of $I_2$ may be at least twice and up to five times that of $I_1$. In an example, $I_2$ may be at least 2 times, 2.1 times, 2.2 times, 2.3 times, 2.4 times, 2.5 times, 2.6 times, 2.7 times, 2.8 times, 2.9 times, 3 times, 3.1 times, 3.2 times, 3.3 times, or 3.4 times that of $I_1$. In another example, $I_2$ may be up to 5 times, 4.9 times, 4.8 times, 4.7 times, 4.6 times, 4.5 times, 4.4 times, 4.3 times, 4.2 times, 4.1 times, 4 times, 3.9 times, 3.8 times, 3.7 times, or 3.6 times that of $I_1$.

A dental implant that satisfies these conditions may exhibit a high initial release of silver, thereby providing antibacterial effects such as bacteria killing and inhibition of bacterial attachment during the early implantation period. In addition, its antibacterial effects may be continuously maintained through a sustained-release effect, in which silver is gradually released over time.

Antibacterial coating method for titanium substrate

The antibacterial coating method for a titanium substrate according to another aspect of the present invention involves forming a silver-titanium oxide matrix on the surface of the titanium substrate and then directly growing apatite on the silver-titanium oxide matrix to maintain osteoconductivity, thereby not only ensuring excellent adhesion between the newly formed bone and the coating layer, but also preventing implant failure caused by bacteria during the early stage of implantation by incorporating silver ions to form a silver-apatite composite.

Specifically, the antibacterial coating method for a titanium substrate according to another aspect of the present invention includes: (a) immersing a titanium substrate in a first solution in which a silver precursor is dissolved so that the Ag concentration is 10 to 300 mM; (b) heating the coating solution to perform hydrothermal treatment and thereby form a silver-titanium oxide matrix on at least a part of the surface of the titanium substrate; and (c) immersing the titanium substrate, on which the silver-titanium oxide matrix has been formed, in a second solution including a calcium precursor, a silver precursor, and a phosphate precursor to form a coating layer including a silver-apatite composite.

Step (a)

The silver precursor included in the first solution may be, for example, one or more selected from the group consisting of silver nitrate ($AgNO_3$), silver perchlorate ($AgClO_4$), silver tetrafluoroborate ($AgBF_4$), silver hexafluorophosphate ($AgPF_6$), silver acetate ($CH_3COOAg$), silver trifluoromethanesulfonate ($AgCF_3SO_3$), silver sulfate ($Ag_2SO_4$), silver 2,4-pentanedionate ($CH_3COCH=COCH_3Ag$), and silver oxides ($AgO$, $Ag_2O$, $Ag_2O_2$, and $Ag_2O_3$), but is not limited thereto.

The Ag concentration of the first solution may be 10 to 300 mM. For example, the concentration may be 10 mM, 11 mM, 12 mM, 13 mM, 14 mM, 15 mM, 16 mM, 17 mM, 18 mM, 19 mM, 20 mM, 21 mM, 22 mM, 23 mM, 24 mM, 25 mM, 26 mM, 27 mM, 28 mM, 29 mM, 30 mM, 31 mM, 32 mM, 33 mM, 34 mM, 35 mM, 36 mM, 37 mM, 38 mM, 39 mM, 40 mM, 41 mM, 42 mM, 43 mM, 44 mM, 45 mM, 46 mM, 47 mM, 48 mM, 49 mM, 50 mM, 51 mM, 52 mM, 53 mM, 54 mM, 55 mM, 56 mM, 57 mM, 58 mM, 59 mM, 60 mM, 61 mM, 62 mM, 63 mM, 64 mM, 65 mM, 66 mM, 67 mM, 68 mM, 69 mM, 70 mM, 71 mM, 72 mM, 73 mM, 74 mM, 75 mM, 76 mM, 77 mM, 78 mM, 79 mM, 80 mM, 81 mM, 82 mM, 83 mM, 84 mM, 85 mM, 86 mM, 87 mM, 88 mM, 89 mM, 90 mM, 91 mM, 92 mM, 93 mM, 94 mM, 95 mM, 96 mM, 97 mM, 98 mM, 99 mM, 100 mM, 110 mM, 120 mM, 130 mM, 140 mM, 150 mM, 160 mM, 170 mM, 180 mM, 190 mM, 200 mM, 210 mM, 220 mM, 230 mM, 240 mM, 250 mM, 260 mM, 270 mM, 280 mM, 290 mM, 300 mM, or a value between any two of these values, but is not limited thereto. When the Ag concentration is below this range, the amount of silver may be insufficient, resulting in reduced antibacterial properties. When the Ag concentration exceeds this range, it may be difficult to form the coating solution or may cause poor coating layer formation due to issues such as viscosity.

Before performing step (a), a pretreatment step may further be included, in which the titanium substrate is treated by at least one method selected from sandblasting, etching, cleaning, and drying, but is not limited thereto.

When the titanium substrate is sandblasted or etched to impart surface roughness, osseointegration properties may be improved, but bacterial attachment may also increase. Foreign substances on the surface may be removed by cleaning or drying the titanium substrate, and the subsequent antibacterial coating layer may be effectively formed.

Step (b)

Step (b) is for heating the coating solution to perform hydrothermal treatment and thereby form a silver-titanium oxide matrix on the surface of the titanium substrate, and may be carried out under conditions of 150 to 300 °C for 1 to 5 hours.

Step (b) may be performed, for example, at 150 °C, 155 °C, 160 °C, 165 °C, 170 °C, 175 °C, 180 °C, 185 °C, 190 °C, 195 °C, 200 °C, 205 °C, 210 °C, 215 °C, 220 °C, 225 °C, 230 °C, 235 °C, 240 °C, 245 °C, 250 °C, 255 °C, 260 °C, 265 °C, 270 °C, 275 °C, 280 °C, 285 °C, 290 °C, 295 °C, 300 °C, or at a value between any two of these values, but is not limited thereto. The temperature conditions of step (b) may affect the amount of silver coating in the silver-titanium oxide matrix and, consequently, antibacterial performance. In addition, step (b) may be performed, for example, for 1 hour, 1.5 hours, 2 hours, 2.5 hours, 3 hours, 3.5 hours, 4 hours, 4.5 hours, 5 hours, or for a value between any two of these values, but is not limited thereto.

Step (c)

The calcium precursor included in the solution in step (c) may be, for example, one or more selected from the group consisting of calcium nitrate ($Ca(NO_3)_2$), calcium carbonate ($CaCO_3$), calcium hydroxide ($Ca(OH)_2$), calcium oxide (CaO), and calcium chloride ($CaCl_2$), and preferably calcium nitrate ($Ca(NO_3)_2$), but is not limited thereto.

The silver precursor included in the solution in step (c) may be, for example, one or more selected from the group consisting of silver nitrate ($AgNO_3$), silver perchlorate ($AgClO_4$), silver tetrafluoroborate ($AgBF_4$), silver hexafluorophosphate ($AgPF_6$), silver acetate ($CH_3COOAg$), silver trifluoromethanesulfonate ($AgCF_3SO_3$), silver sulfate ($Ag_2SO_4$), silver 2,4-pentanedionate ($CH_3COCH=COCH_3Ag$), and silver oxides (AgO, $Ag_2O$, $Ag_2O_2$, and $Ag_2O_3$), and preferably silver nitrate ($AgNO_3$), but is not limited thereto.

The phosphate precursor included in the solution in step (c) may be one or more selected from the group consisting of diammonium phosphate, disodium phosphate, and dipotassium phosphate, but is not limited thereto.

In step (c), the Ag/Ca molar ratio of the solution may be 0.3 to 0.6, and the Ca/P molar ratio may be 0.9 to 1.1. Since the ranges of the Ag/Ca and Ca/P molar ratios overlap those of the coating layer of the dental implant described above, a detailed description thereof will be omitted.

The temperature and time conditions of step (c) may affect the coating amount of the coating layer including the silver-apatite composite and, consequently, osseointegration.

Step (c) may be performed at a temperature of 10 to 40 °C. For example, it may be performed at 10 °C, 11 °C, 12 °C, 13 °C, 14 °C, 15 °C, 16 °C, 17 °C, 18 °C, 19 °C, 20 °C, 21 °C, 22 °C, 23 °C, 24 °C, 25 °C, 26 °C, 27 °C, 28 °C, 29 °C, 30 °C, 31 °C, 32 °C, 33 °C, 34 °C, 35 °C, 36 °C, 37 °C, 38 °C, 39 °C, 40 °C, or at a value between any two of these values, but is not limited thereto. When the temperature is too low, it may take a long time to form the coating layer. On the other hand, when the temperature is too high, delamination may occur, causing the coating layer with attached bone to separate from the implant surface.

Step (c) may be performed for 40 to 180 minutes. For example, it may be performed for 40 minutes, 41 minutes, 42 minutes, 43 minutes, 44 minutes, 45 minutes, 46 minutes, 47 minutes, 48 minutes, 49 minutes, 50 minutes, 51 minutes, 52 minutes, 53 minutes, 54 minutes, 55 minutes, 56 minutes, 57 minutes, 58 minutes, 59 minutes, 60 minutes, 61 minutes, 62 minutes, 63 minutes, 64 minutes, 65 minutes, 66 minutes, 67 minutes, 68 minutes, 69 minutes, 70 minutes, 71 minutes, 72 minutes, 73 minutes, 74 minutes, 75 minutes, 76 minutes, 77 minutes, 78 minutes, 79 minutes, 80 minutes, 81 minutes, 82 minutes, 83 minutes, 84 minutes, 85 minutes, 86 minutes, 87 minutes, 88 minutes, 89 minutes, 90 minutes, 91 minutes, 92 minutes, 93 minutes, 94 minutes, 95 minutes, 96 minutes, 97 minutes, 98 minutes, 99 minutes, 100 minutes, 110 minutes, 120 minutes, 130 minutes, 140 minutes, 150 minutes, 160 minutes, 170 minutes, 180 minutes, or for a value between any two of these values, but is not limited thereto. When the coating time is too short, the coating may not be properly applied to the implant or the adhesion of the coating layer may be reduced. On the other hand, when the coating time is too long, the coating layer may become excessively thick, which may cause implant failure due to delamination of the coating layer.

After step (c), post-treating the titanium substrate on which the coating layer is formed by one or more methods selected from the group consisting of ethanol immersion and ultrasonic cleaning may be further included. In this case, such post-treatment may be advantageous for osseointegration and/or antibacterial properties, but is not limited thereto.

Hereinafter, the examples of this specification will be described in more detail. However, the following experimental results only describe representative outcomes among the above examples, and the scope and content of this specification cannot be construed as being reduced or limited by the examples. The effects of various embodiments of this specification that are not explicitly presented below will be specifically described in the relevant sections.

Example 1

A disk-shaped (cylindrical) Cp-Ti (Titanium Grade 4) specimen with a diameter of 6 mm, a thickness of 2 mm, and a length of 10 mm was prepared. After SLA pretreatment (a surface treatment method involving aluminum blasting followed by acid etching), the specimen was immersed in a 12% nitric acid ($HNO_3$) solution for 5 minutes, subjected to ultrasonic cleaning, and then rinsed with distilled water to remove residual acid from the surface. The 12% nitric acid ($HNO_3$) solution was prepared by mixing a 60% nitric acid solution and distilled water in a 1:4 ratio, and the ultrasonic cleaning was performed for 15 minutes.

After dissolving calcium nitrate, silver nitrate, and diammonium hydrogen phosphate in distilled water to prepare a solution, the pretreated titanium specimen was immersed in this solution (at a temperature of 22 °C) for 70 minutes to prepare a dental implant specimen with a coating layer having an Ag/Ca molar ratio of 0.3 and a Ca/P molar ratio of 1.

Example 2

A dental implant specimen with a coating layer was prepared in the same manner as in Example 1, except that the Ag/Ca molar ratio was 0.4.

Example 3

A dental implant specimen with a coating layer was prepared in the same manner as in Example 1, except that the Ag/Ca molar ratio was 0.5.

Comparative Example 1

A dental implant specimen with a coating layer was prepared in the same manner as in Example 1, except that silver nitrate was not used in the preparation of the solution, resulting in an Ag/Ca molar ratio of 0.

Comparative Example 2

A dental implant specimen with a coating layer was prepared in the same manner as in Example 1, except that the Ag/Ca molar ratio was 0.1.

Comparative Example 3

A dental implant specimen with a coating layer was prepared in the same manner as in Example 1, except that the Ag/Ca molar ratio was 0.2.

Experimental Example 1: Evaluation of antibacterial properties

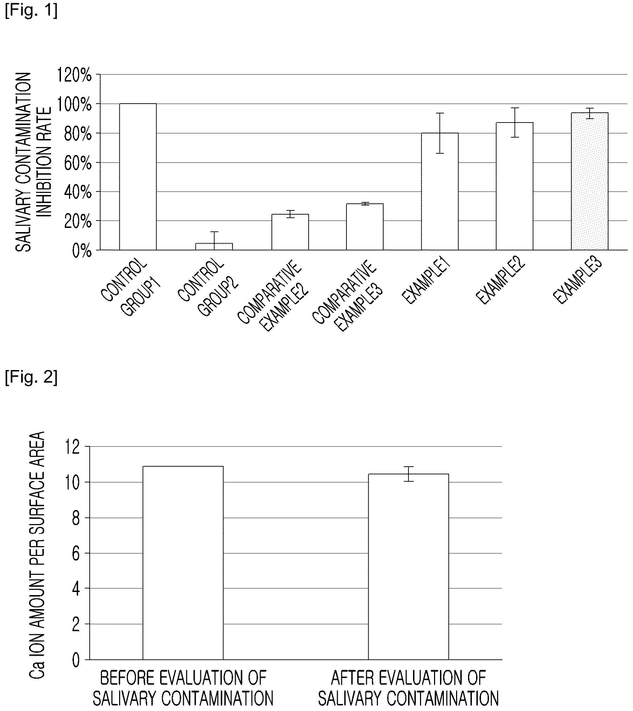

For the evaluation of antibacterial properties, the inhibition rate of salivary contamination was measured. The measurement of the salivary contamination inhibition rate was carried out as follows, and the results are shown in FIG. 1.

(1) To minimize the effect of saliva viscosity, saliva was directly collected and mixed with PBS at a 1:1 ratio, and the mixture was shaken thoroughly.
(2) The mixture was uniformly dispensed in a well plate, and each implant was independently immersed in each well for 5 seconds.
(3) After removing each implant from the well plate, it was placed in an individual bacterial culture solution (for each implant) and incubated for bacterial culture at 37 °C for 4 hours.
(4) The cultured bacteria were sampled, and the level of bacterial growth was compared using a UV-vis spectrophotometer.
(5) The negative control group (SA alpha) was a specimen in which the saliva prepared in step (1) was added to the bacterial culture solution, and was set as the standard for a salivary contamination inhibition rate of 0% (100% contamination rate).
(6) The positive control group (Control) refers to the optical density of the bacterial culture solution without saliva contamination, and was set as the standard for a salivary contamination inhibition rate of 100% (0% contamination rate).

Referring to FIG. 1, it can be seen that Examples 1 to 3 exhibited excellent antibacterial properties, with a salivary contamination inhibition rate of 80% or higher. In particular, Example 3 exhibited a salivary contamination inhibition rate of 90% or higher, demonstrating outstanding antibacterial properties.

Experimental Example 2: Measurement of Ag-apatite change amount after evaluation of antibacterial properties

The amount of Ca ions in Example 2 was measured before and after the evaluation of antibacterial properties to determine the residual amount of apatite, and the results are shown in FIG. 2. Referring to FIG. 2, it can be seen that a substantial amount of apatite remained on the titanium substrate surface even after the evaluation of salivary contamination. This is because the suppression of biofilm formation by the silver-apatite composite controls the surrounding acidification. Therefore, it can be expected that excellent osseointegration will be maintained even after the evaluation of antibacterial properties.

Experimental Example 3: Ag-apatite FE-SEM analysis

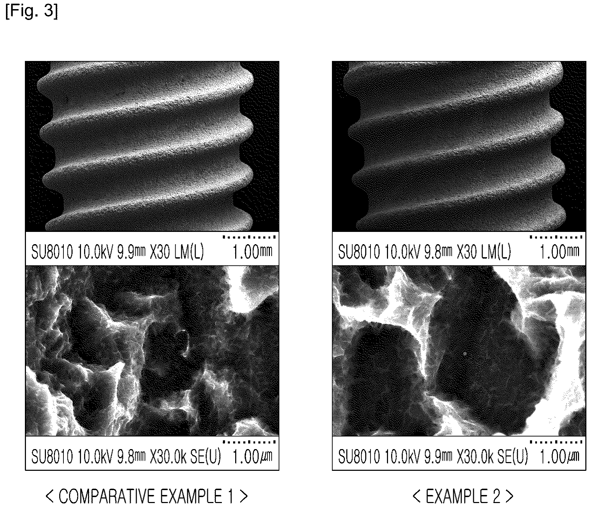

Surface analysis using SEM was conducted on Example 2 and Comparative Example 1, and the results are shown in FIG. 3. FIG. 3A shows Comparative Example 1, and FIG. 3B shows Example 2.

Referring to FIG. 3, it can be seen that Example 2 had the same structure and crystallinity as Comparative Example 1, which does not include silver.

Experimental Example 4: Ag-apatite EDS analysis

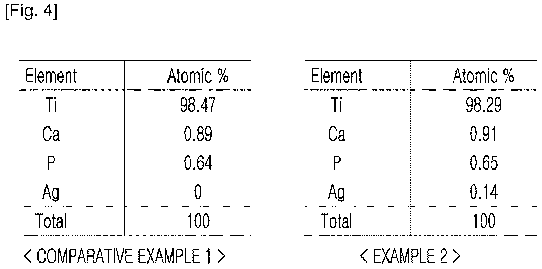

EDS analysis was conducted to confirm the distribution of Ag ions on the surfaces of Example 2 and Comparative Example 1, and the results are shown in FIG. 4.

Referring to FIG. 4, it can be seen that Example 2 exhibited a uniform distribution of silver on the surface of the titanium substrate. FIG. 4A shows Comparative Example 1, and FIG. 4B shows Example 2.

Experimental Example 5: Ag-apatite XPS analysis

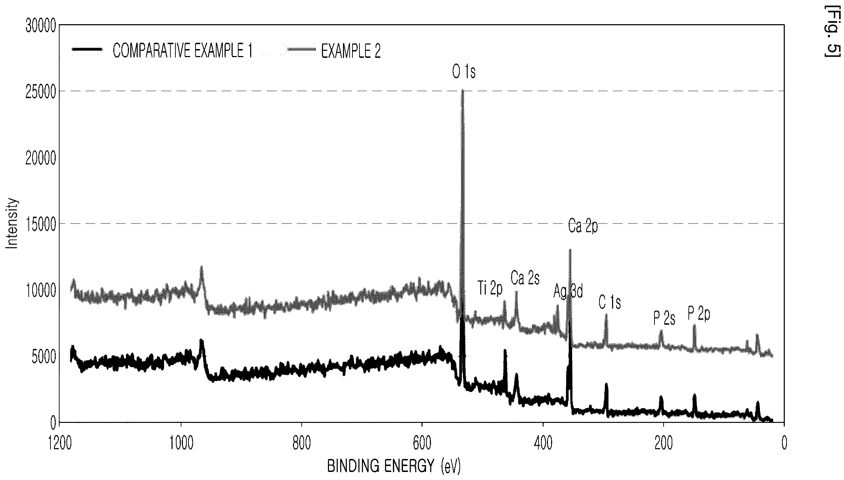

XPS analysis was conducted to analyze the atomic states of Example 2 and Comparative Example 1, and the results are shown in FIG. 5.

Referring to FIG. 5, the analysis graph of Example 2 revealed a distinct Ag ion (Ag 3d) peak. Accordingly, in Example 2, it can be seen that the titanium substrate surface was effectively coated with silver, and that silver was present in the form of a silver-apatite composite by partially replacing calcium within apatite. In addition, the absence of an Ag 2p peak demonstrates that silver was not present in the form of metallic silver.

Experimental Example 6: Selection of antibacterial ions

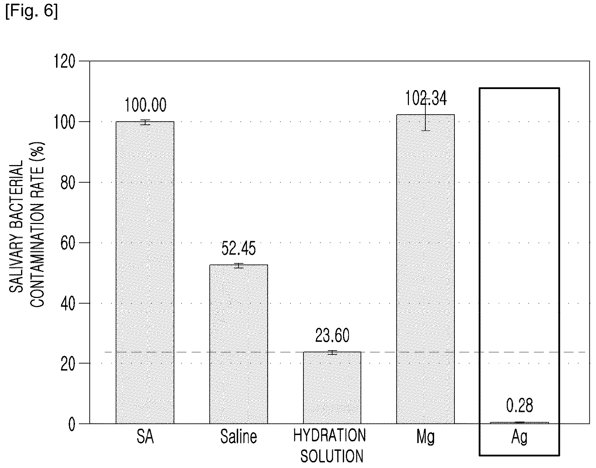

To select the antibacterial ion, the contamination rate of salivary bacteria was evaluated. Saliva (SA) was used as the control group, and for the experimental groups, 100 mL each of saline, a hydration solution (EC), a magnesium ion salt, and a silver ion salt were prepared. Into each control and experimental solution, 1 mL of a suspension of salivary bacteria isolated from prepared saliva was added and maintained at 37 °C under anaerobic conditions for 16 hours. After 16 hours, the contamination rate of salivary bacteria was measured, and the results are shown in FIG. 6. The contamination rate of salivary bacteria in the control group was set to 100%, and the contamination rates in the experimental groups were measured accordingly.

Referring to FIG. 6, the contamination rates in the saline and hydration solution (EC) were 52.45% and 23.6%, respectively, compared to saliva. The magnesium ion salt, which is an antibacterial ion salt, showed the highest contamination rate at 102.34%. In contrast, the silver ion salt showed a saliva bacterial contamination rate of 0.28%, indicating a 99.7% reduction in bacterial contamination compared to saliva. Therefore, silver ions were selected as the antibacterial ion.

Experimental Example 7: Selection of silver-titanium oxide matrix

(1) Selection of silver-titanium oxide matrix formation method

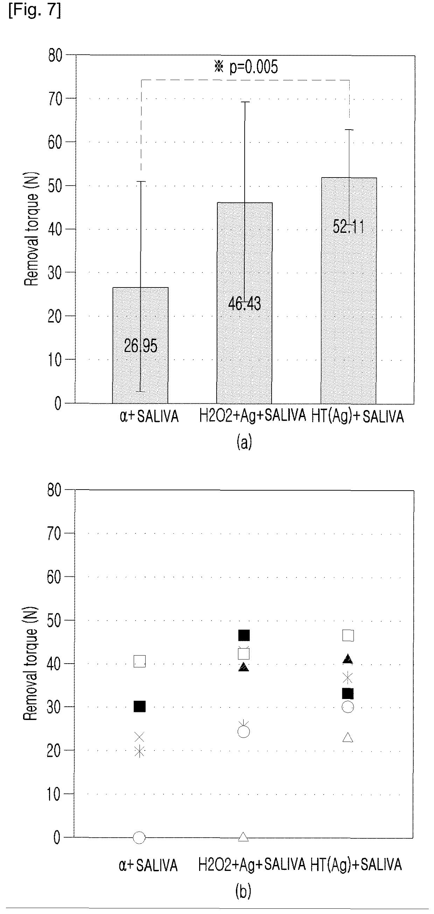

To select the method for forming the silver-titanium oxide matrix, an animal experiment was conducted. A titanium implant was prepared as the control group. For the experimental groups, titanium implants treated with a solution containing a silver ion salt and hydrogen peroxide, as well as titanium implants hydrothermally treated with a solution containing a silver ion salt, were prepared. Eight samples were prepared for each implant group. Before implantation, the prepared implants were contaminated by immersing them in saliva for 24 hours, and then implanted into the tibia of rabbits and maintained for 21 days to allow bone formation. After this period, the removal torque was evaluated. The results of the experiment are shown in FIG. 7.

FIG. 7A shows the average removal torque values for the eight samples in each group. FIG. 7B shows the individual removal torque values for each of the eight samples. For untreated titanium implants, the average removal torque was 26.95 N, which was the lowest among the groups, and two out of eight implants failed to achieve stable implantation. On the other hand, the implants coated with silver ions showed removal torque values of 46.43 N and 52.11 N, respectively, confirming that silver ion treatment reduced contamination from saliva. In addition, implants coated with silver ions by hydrothermal treatment exhibited the highest removal torque and had zero failures out of eight implants. Therefore, the excellent antibacterial properties of silver ions against salivary bacteria were confirmed, and the hydrothermal method was selected as the coating method.

(2) Selection of hydrothermal treatment conditions 1

To determine the hydrothermal treatment conditions, antibacterial performance was evaluated according to temperature, time, and silver ion concentration.

For the evaluation, nine titanium substrates were prepared as samples, each with a size of 3 x 3 cm$^2$. The hydrothermal treatment conditions for each titanium substrate were as follows: temperatures of 180 °C, 200 °C, and 250 °C; times of 1 hour, 2 hours, and 3 hours; and silver ion concentrations of 30 mM, 60 mM, and 100 mM.

As salivary bacteria, *Aggregatibacter actinomycetemcomitans* (ATCC 33384) and *Porphyromonas gingivalis*, which are causative agents of acute periodontitis or peri-implantitis, were used. Each type of prepared salivary bacteria was inoculated into BHI (brain heart infusion broth, Merck) medium and cultured at 37 °C under anaerobic conditions for 16 to 24 hours. The cultured solution was diluted with BHI medium until the optical density at 600 nm reached 0.1, and further diluted with BHI medium so that the bacterial concentration became 1/10. Through this process, two types of salivary bacterial suspensions were prepared.

The nine titanium substrates were sterilized by UV exposure for 30 minutes on a clean bench, and then one per well was placed in a 24-well plate. 1 mL of the prepared salivary bacterial suspension was added to each well, and the plates were incubated at 37 °C under anaerobic conditions for 16 hours. In a new 24-well plate, 1 mL of 0.1 wt% crystal violet solution was added to each well. The samples were transferred into the wells containing crystal violet and incubated at room temperature for 10 minutes to stain the bacteria purple.

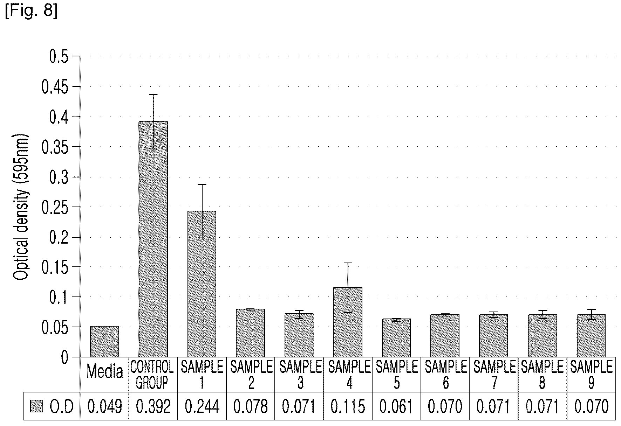

A new 24-well plate was prepared by adding 1 mL of phosphate-buffered saline (PBS) to each well. The stained samples were transferred into these wells, and the above process was repeated once more. After PBS washing, samples were placed on dry wipes to blot the bottom surfaces. A new 24-well plate was loaded with 500 μL of 30 wt% acetic acid solution per well. The stained samples were then transferred into these wells and incubated at room temperature for 20 minutes to dissolve the dye. 200 μL of each of the dissolved solutions was input to a 96-well plate, and the optical density at 595 nm was measured using a microplate reader. The results are shown in FIG. 8. For comparison of test samples, control groups including bacterial-free media and untreated titanium substrate samples were subjected to the salivary bacterial suspension in the same manner as described above, and their optical density (O.D. at 595 nm) was measured.

The hydrothermal treatment conditions for the nine samples are shown in Table 1 below.

[Table 1]

| Classification | Temperature (°C) | Time (h) | Silver ion concentration (mM) | Optical density (595 nm) |
|---|---|---|---|---|
| Sample 1 | 180 | 1 | 30 | 0.244 |
| Sample 2 | 180 | 2 | 60 | 0.078 |
| Sample 3 | 180 | 3 | 100 | 0.071 |
| Sample 4 | 200 | 1 | 60 | 0.115 |
| Sample 5 | 200 | 2 | 100 | 0.061 |
| Sample 6 | 200 | 3 | 30 | 0.070 |
| Sample 7 | 250 | 1 | 100 | 0.071 |
| Sample 8 | 250 | 2 | 30 | 0.071 |
| Sample 9 | 250 | 3 | 60 | 0.070 |

Referring to Table 1 and FIG. 8, all samples except Samples 1 and 4 exhibited optical density (O.D. at 595 nm) values of 0.08 or less, demonstrating the excellent inhibitory effect of silver ions on salivary bacteria.

To determine the optimal hydrothermal treatment conditions, O.D. (595 nm) values were summed according to temperature, time, and silver ion concentration, and the results are shown in Table 2.

[Table 2]

| Classification | Condition | Optical density (595 nm) | Difference (maximum value - minimum value) |
|---|---|---|---|
| Temperature (°C) | 180 | 0.393 | 0.181 |
| | 200 | 0.246 | |
| | 250 | 0.212 | |

(continued)

| Classification | Condition | Optical density (595 nm) | Difference (maximum value - minimum value) |
|---|---|---|---|
| Time (h) | 1 | 0.430 | 0.22 |
| | 2 | 0.210 | |
| | 3 | 0.211 | |
| Silver ion concentration (mM) | 30 | 0.385 | 0.182 |
| | 60 | 0.263 | |
| | 100 | 0.203 | |

Referring to Table 2, the optical density (O.D. at 595 nm) values for hydrothermal treatment at 200 °C and 250 °C were 0.246 and 0.212, respectively, indicating improved antibacterial properties compared to treatment at 180 °C. Additionally, with respect to treatment time, superior results were observed under the conditions of 2 hours and 3 hours. For silver ion concentration, the contamination rate was notably reduced at concentrations of 60 mM and 100 mM. Therefore, the optimal hydrothermal treatment conditions for enhancing antibacterial properties were determined to be a temperature of 200 to 250 °C, a time of 2 to 3 hours, and a silver ion concentration of 60 to 100 mM.

(3) Selection of hydrothermal treatment conditions 2

To determine the hydrothermal treatment conditions, the inhibition rate of salivary bacterial contamination, the initial titanium (Ti) ion elution amount, and the silver (Ag) ion release amount were evaluated according to the $H_2O_2$ concentration (chemical treatment), temperature, and time during hydrothermal treatment.

For the evaluation, nine titanium substrates were prepared as samples, each with a size of 3 x 3 $cm^2$. The hydrothermal treatment conditions for each titanium substrate were as follows: $H_2O_2$ concentrations of 0 wt% (untreated), 1 wt%, and 2 wt%; temperatures of 180 °C, 200 °C, and 250 °C; times of 1 hour, 2 hours, and 3 hours; and the silver ion concentration fixed at 100 mM.

The inhibition rate of salivary bacterial contamination was measured in the same manner as the antibacterial performance evaluation in Experimental Example 1, and the inhibition rate of salivary bacterial contamination was derived based on the O.D. (595 nm) of untreated titanium substrate samples contaminated with salivary bacteria.

Inhibition rate of salivary bacterial contamination (%) = (Optical density value of antibacterial-coated sample under hydrothermal treatment condition) / (Optical density value of untreated sample)

To derive the optimal hydrothermal treatment conditions, the sum of each of the inhibition rate of salivary bacterial contamination, the initial Ti ion elution amount, and the Ag ion release amount according to each $H_2O_2$ concentration, temperature, and time are shown in Table 3.

[Table 3]

| Classification | Condition | Inhibition rate of salivary bacterial contamination (%) Sum | Difference (maximum value - minimum value) | Ag release amount (ppb) Sum | Difference (maximum value - minimum value) | Initial Ti elution amount (ppb) Sum | Difference (maximum value - minimum value) |
|---|---|---|---|---|---|---|---|
| $H_2O_2$ concentration | 0% | 2.89 | 0.08 | 1517.42 | 772.7 | 10.32 | 668.03 |
| | 1% | 2.96 | | 1774.65 | | 178.16 | |
| | 2% | 2.97 | | 2290.12 | | 678.35 | |
| Temperature | 150 °C | 2.93 | 0.04 | 947.67 | 2323.8 | 689.47 | 658.26 |
| | 200 °C | 2.93 | | 3271.52 | | 31.21 | |
| | 250 °C | 2.97 | | 1363 | | 146.15 | |
| Time | 1 h | 2.92 | 0.06 | 1675.6 | 463.3 | 179.19 | 394.58 |
| | 2 h | 2.93 | | 2138.9 | | 541.11 | |
| | 3 h | 2.98 | | 1767.69 | | 146.53 | |

Referring to Table 3, the inhibition rate of salivary bacterial contamination according to $H_2O_2$ concentration conditions during hydrothermal treatment showed only a minor difference, with a maximum-minimum difference of 0.08, but it was observed that the initial Ti ion elution amount increased significantly as the $H_2O_2$ concentration increased. Accordingly, $H_2O_2$ was excluded in the hydrothermal treatment. In addition, under temperature conditions of 200 °C or higher, the Ag ion release amount was superior, while the initial Ti ion elution was lower, resulting in meaningful outcomes. Regarding treatment time, the inhibition rate of salivary bacterial contamination was highest at 3 hours, the Ag ion release amount was greatest at 2 hours, and the initial Ti ion elution amount was lowest at 3 hours.

(4) FE-SEM and EDS analyses of silver-titanium oxide matrix

Based on the results of the antibacterial ion selection and the selection of the method and conditions for forming the silver-titanium oxide matrix, a titanium substrate with a silver-titanium oxide matrix (Preparation Example 1) was prepared.

Specifically, a Cp-Ti (Titanium Grade 4) specimen in the form of a disk (cylinder) with a diameter of 6 mm, a thickness of 2 mm, and a length of 10 mm was prepared. The specimen was immersed in a silver nitrate solution with an Ag concentration of 100 mM and then subjected to hydrothermal treatment by heating at 200 °C for 3 hours. After hydrothermal treatment, the specimen was immersed in deionized water for 5 minutes and washed, thereby preparing a titanium substrate with a silver-titanium oxide matrix (Preparation Example 1) on its surface.

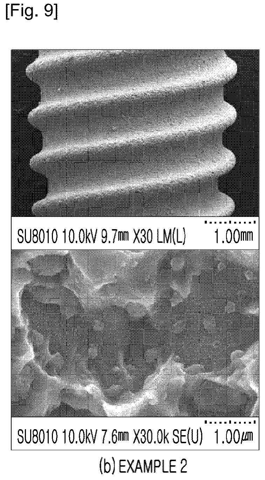

Subsequently, FE-SEM and EDS analyses were conducted to analyze the surface of Preparation Example 1, and the results are shown in FIGS. 9 to 11.

FIG. 9 shows FE-SEM images of Preparation Example 1. Referring to FIG. 9, it can be seen that silver particles were formed on the surface of Preparation Example 1.

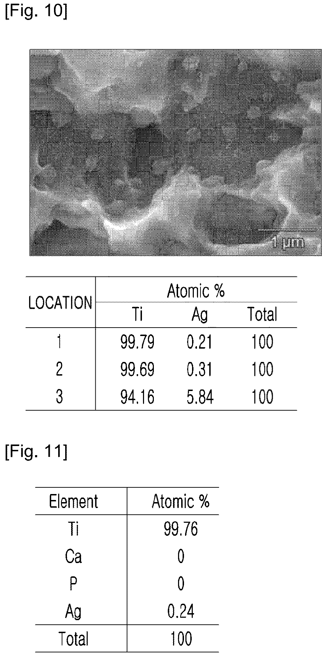

FIG. 10 presents the elemental distribution analysis of titanium and silver by location in the SEM image of FIG. 9. Referring to FIG. 10, location 1 showed 99.79 at% titanium and 0.21 at% silver, location 2 showed 99.69 at% titanium and 0.31 at% silver, and location 3 showed 94.16 at% titanium and 5.84 at% silver. At location 3, the formation of bulk silver particles was observed, resulting in an increased silver atomic percentage.

FIG. 11 shows the EDS image of Preparation Example 1. Referring to FIG. 11, it can be seen that silver was uniformly distributed on the surface of the titanium substrate in Preparation Example 1.

(5) XPS analysis of silver-titanium oxide matrix

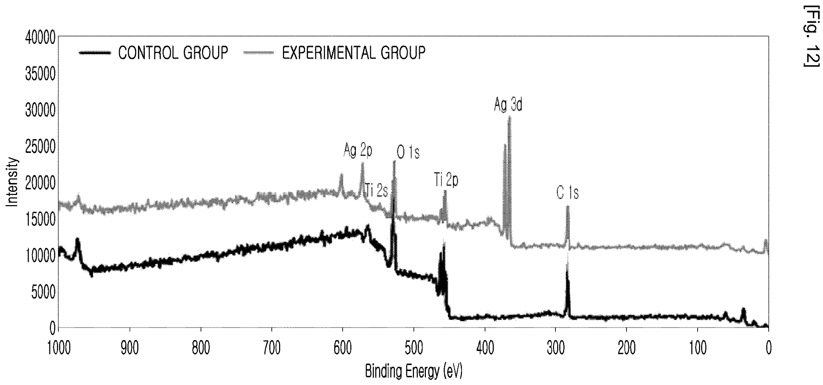

XPS analysis was conducted to analyze the atomic states of the silver-titanium oxide matrix, and the results are shown in FIG. 12. An untreated Cp-Ti (Titanium Grade 4) specimen as the control group was also subjected to XPS analysis.

Referring to FIG. 12, the metallic silver (Ag 2p) and oxidized silver ion (Ag 3d) peaks were simultaneously detected in the analysis graph of the silver-titanium oxide matrix. This demonstrates that the surface of the titanium substrate was effectively coated with silver particles. In the control group, the absence of surface treatment resulted in faint oxidized Ti ion peaks.

Comparing the peak intensities of metallic Ag and oxidized Ag ions, the Ag 3d peak at 360 to 375 eV was approximately 3.5 times higher than the Ag 2p peak at 570 to 580 eV.

Experimental Example 8: FE-SEM analysis of dental implant specimens according to apatite coating layer

Example 4

A dental implant specimen with a coating layer having an Ag/Ca molar ratio of 0.4 and a Ca/P molar ratio of 1 was prepared by preparing a solution by dissolving calcium nitrate, silver nitrate, and diammonium hydrogen phosphate in distilled water, and then immersing the titanium specimen from Preparation Example 1 in the solution (at a temperature of 22 °C) for 70 minutes.

Comparative Example 4

A dental implant specimen with a coating layer was prepared in the same manner as in Example 4, except that the Ag/Ca molar ratio was 0.67, and the Ca/P molar ratio was 0.6.

Comparative Example 5

A dental implant specimen with a coating layer was prepared in the same manner as in Example 4, except that silver nitrate was not used in the preparation of the solution, resulting in an Ag/Ca molar ratio of 0.

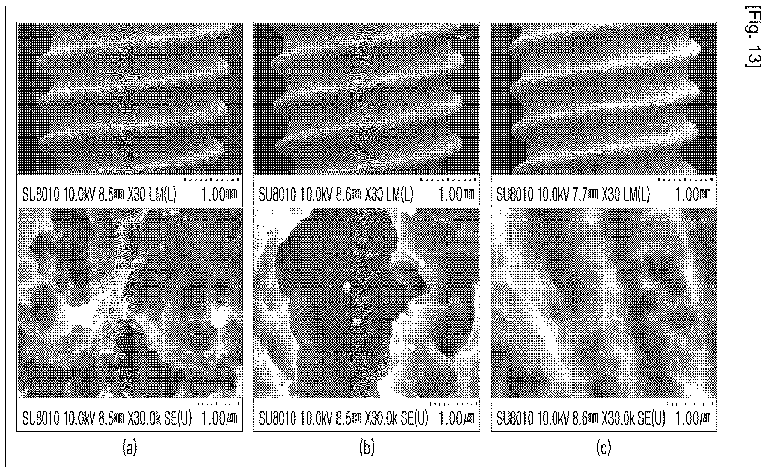

FE-SEM analysis was conducted on the surfaces of the specimens from Example 4, Comparative Example 4, and Comparative Example 5, and the results are shown in FIG. 13.

FIG. 13A shows FE-SEM images of Example 4, FIG. 13B shows FE-SEM images of Comparative Example 4, and FIG. 13C shows FE-SEM images of Comparative Example 5. Referring to FIG. 13, it can be seen that the coating layer was effectively formed in Example 4. On the other hand, overcoating was observed in Comparative Example 4, and in Comparative Example 5, no coating layer was observed.

Experimental Example 9: Evaluation of Ag release amount

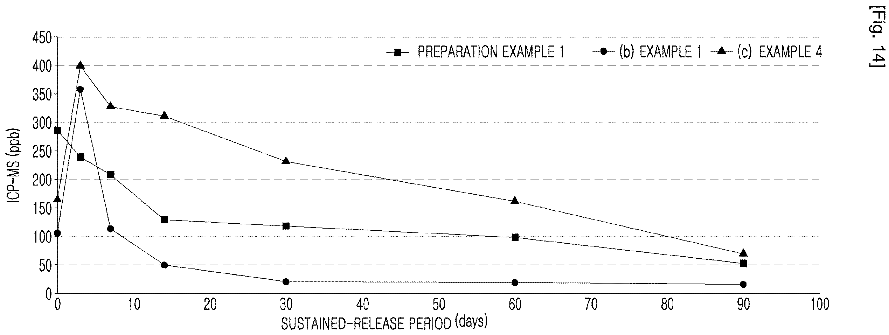

To evaluate the amount of Ag released according to the immersion period, specimens from Preparation Example 1, Example 1, and Example 4 were immersed in deionized water (DW), and the amount of Ag released over 90 days was measured. The Ag release amount was measured using an ICP-MS analyzer, and the results are shown in FIG. 14.

Referring to FIG. 14, FIG. 14A represents Preparation Example 1, FIG. 14B represents Example 1, and FIG. 14C represents Example 4. Immediately after immersion, the Ag ion release amount was 150 ppb or more, and after 3 days of immersion, the Ag ion release amount reached approximately 400 ppb. After 3 days of immersion, the Ag ion release amount gradually decreased, and after 30 days, 200 ppb or more of Ag ions were released in a sustained manner. After 60 days of immersion, the Ag ion release amount was 150 ppb or more, and after 90 days of immersion, the Ag ion release amount was 50 ppb or more. Therefore, when Example 4 of the present invention is applied to dental implants, it can be expected that the antibacterial ions, silver ions, will be released in a sustained manner during the initial implantation period (about 3 months), and the early failure rate of implantation will be significantly reduced.

Experimental Example 10: Evaluation of antibacterial performance according to Ag ion release period

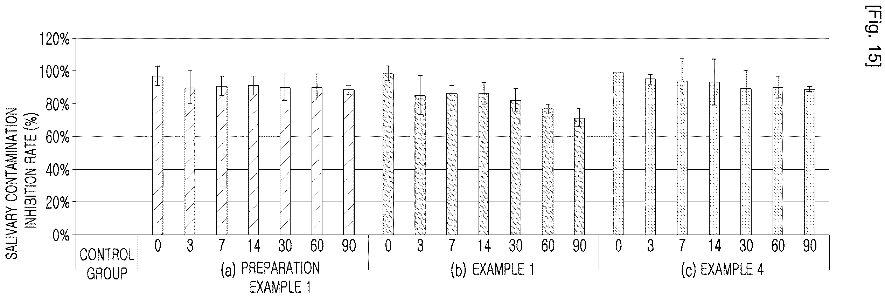

To evaluate the antibacterial performance according to the Ag ion release period, the inhibition rate of salivary bacterial contamination was measured for specimens from Preparation Example 1, Example 1, and Example 4, and the results are shown in FIG. 15.

Referring to FIG. 15, it was observed that in Example 4, the inhibition rate of salivary bacterial contamination was 90% or more even after 3 days of immersion. Although the inhibition rate of salivary bacterial contamination gradually decreased over the 90-day period, it maintained a level of 80% or more. These results indicate that Ag ions are released in a sustained manner for up to 90 days.

The description of this specification described above is for illustrative purposes, and those skilled in the art will understand that the present invention can be easily modified into other specific forms without changing the technical idea or essential features of this specification. Therefore, the embodiments described above should be understood in all respects as illustrative and not restrictive. For example, each component described as single may be implemented in a distributed form, and likewise, each component described as distributed may be implemented in a combined form.

The scope of this specification is indicated by the claims described below, and all changes or modified forms derived from the meaning and the scope of the claims and their equivalent concepts should be construed as being included in the scope of the present invention.

**Claims**

1. A dental implant comprising:

   a titanium substrate; and
   a coating layer formed on at least a part of a surface of the titanium substrate and including a silver-apatite composite,
   wherein the coating layer has an Ag/Ca molar ratio of 0.3 to 0.6 and a Ca/P molar ratio of 0.9 to 1.1.

2. The dental implant of claim 1, wherein the coating layer is composed of apatite and a silver-apatite composite.

3. The dental implant of claim 1, wherein the coating layer does not include metallic silver.

4. The dental implant of claim 1, wherein the coating layer has an average thickness of 50 nm or less.

5. An antibacterial coating method for a titanium substrate, comprising:

(a) preparing a solution including a calcium precursor, a silver precursor, and a phosphate precursor; and
(b) immersing a titanium substrate in the solution to form a coating layer including a silver-apatite composite on a surface of the titanium substrate,

wherein the solution has an Ag/Ca molar ratio of 0.3 to 0.6 and a Ca/P molar ratio of 0.9 to 1.1.

6. The antibacterial coating method of claim 5, wherein the calcium precursor is one or more selected from the group consisting of calcium nitrate ($Ca(NO_3)_2$), calcium carbonate ($CaCO_3$), calcium hydroxide ($Ca(OH)_2$), calcium oxide (CaO), and calcium chloride ($CaCl_2$).

7. The antibacterial coating method of claim 5, wherein the silver precursor is one or more selected from the group consisting of silver nitrate ($AgNO_3$), silver perchlorate ($AgClO_4$), silver tetrafluoroborate ($AgBF_4$), silver hexafluor-ophosphate ($AgPF_6$), silver acetate ($CH_3COOAg$), silver trifluoromethanesulfonate ($AgCF_3SO_3$), silver sulfate ($Ag_2SO_4$), silver 2,4-pentanedionate ($CH_3COCH=COCH_3Ag$), and silver oxides (AgO, $Ag_2O$, $Ag_2O_2$, and $Ag_2O_3$).

8. The antibacterial coating method of claim 5, wherein the phosphate precursor is one or more selected from the group consisting of diammonium phosphate, disodium phosphate, and dipotassium phosphate.

9. The antibacterial coating method of claim 5, further comprising pretreating the titanium substrate by one or more methods selected from the group consisting of sandblasting, anodizing, acid or base treatment, heat treatment, UV treatment, and plasma treatment, before step (b).

10. The antibacterial coating method of claim 5, wherein step (b) is performed at a temperature of 10 to 40 °C for 40 to 180 minutes.

11. The antibacterial coating method of claim 5, further comprising post-treating the titanium substrate on which the coating layer is formed by one or more methods selected from the group consisting of immersion in ethanol and ultrasonic cleaning, after step (b).

[Fig. 1]

[Fig. 2]

[Fig. 3]

SU8010 10.0kV 9.9mm X30 LM(L)   1.00mm

SU8010 10.0kV 9.8mm X30.0k SE(U)  1.00μm

< COMPARATIVE EXAMPLE 1 >

SU8010 10.0kV 9.8mm X30 LM(L)   1.00mm

SU8010 10.0kV 9.9mm X30.0k SE(U)  1.00μm

< EXAMPLE 2 >

[Fig. 4]

| Element | Atomic % |
|---------|----------|
| Ti | 98.47 |
| Ca | 0.89 |
| P | 0.64 |
| Ag | 0 |
| Total | 100 |

< COMPARATIVE EXAMPLE 1 >

| Element | Atomic % |
|---------|----------|
| Ti | 98.29 |
| Ca | 0.91 |
| P | 0.65 |
| Ag | 0.14 |
| Total | 100 |

< EXAMPLE 2 >

[Fig. 5]

[Fig. 6]

[Fig. 7]

(a)

(b)

[Fig. 8]

| | Media | CONTROL GROUP | SAMPLE 1 | SAMPLE 2 | SAMPLE 3 | SAMPLE 4 | SAMPLE 5 | SAMPLE 6 | SAMPLE 7 | SAMPLE 8 | SAMPLE 9 |
|---|---|---|---|---|---|---|---|---|---|---|---|
| O.D | 0.049 | 0.392 | 0.244 | 0.078 | 0.071 | 0.115 | 0.061 | 0.070 | 0.071 | 0.071 | 0.070 |

[Fig. 9]

SU8010 10.0kV 9.7mm X30 LM(L)    1.00mm

SU8010 10.0kV 7.6mm X30.0k SE(U)  1.00μm

(b) EXAMPLE 2

[Fig. 10]

| LOCATION | Atomic % | | |
|----------|-------|------|-------|
|          | Ti    | Ag   | Total |
| 1        | 99.79 | 0.21 | 100   |
| 2        | 99.69 | 0.31 | 100   |
| 3        | 94.16 | 5.84 | 100   |

[Fig. 11]

| Element | Atomic % |
|---------|----------|
| Ti      | 99.76    |
| Ca      | 0        |
| P       | 0        |
| Ag      | 0.24     |
| Total   | 100      |

[Fig. 12]

[Fig. 13]

[Fig. 14]

[Fig. 15]

# EP 4 643 812 A1

## INTERNATIONAL SEARCH REPORT

| | International application No. |
|---|---|
| | **PCT/KR2023/021640** |

### A. CLASSIFICATION OF SUBJECT MATTER

**A61C 8/00**(2006.01)i; **A61L 27/32**(2006.01)i; **A61L 27/06**(2006.01)i; **A61L 27/30**(2006.01)i; **A01N 59/06**(2006.01)i; **A01N 59/16**(2006.01)i; **A01N 59/26**(2006.01)i; **A01N 59/08**(2006.01)i

According to International Patent Classification (IPC) or to both national classification and IPC

### B. FIELDS SEARCHED

Minimum documentation searched (classification system followed by classification symbols)

A61C 8/00(2006.01); A61L 27/04(2006.01); A61L 27/06(2006.01); A61L 27/28(2006.01); A61L 27/30(2006.01); A61L 27/32(2006.01); A61L 27/54(2006.01); A61L 27/56(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 치과용 임플란트(dental implant), 항균코팅(antibacterial coating), 골유착성 (osteointegration property), 항균성(antibacterial property), 은-아파타이트 복합체(silver-apatite complex)

### C. DOCUMENTS CONSIDERED TO BE RELEVANT

| Category* | Citation of document, with indication, where appropriate, of the relevant passages | Relevant to claim No. |
|---|---|---|
| X | JP 2019-150648 A (SMITH & NEPHEW INC.) 12 September 2019 (2019-09-12) See paragraphs [0013]-[0034], [0040]-[0043], [0086], [0092], [0114] and [0121]. | 1-8,10 |
| Y | | 9,11 |
| Y | KR 10-2009-0017693 A (ACCENTUS PLC) 18 February 2009 (2009-02-18) See paragraphs [0008]-[0012] and [0018]-[0023]. | 9,11 |
| A | US 2018-0289858 A1 (THE UNIVERSITY OF TOLEDO) 11 October 2018 (2018-10-11) See entire document. | 1-11 |
| A | KR 10-2012-0075202 A (RESEARCH INSTITUTE OF INDUSTRIAL SCIENCE & TECHNOLOGY) 06 July 2012 (2012-07-06) See entire document. | 1-11 |

☑ Further documents are listed in the continuation of Box C.   ☑ See patent family annex.

| | |
|---|---|
| \* Special categories of cited documents: | "T" later document published after the international filing date or priority date and not in conflict with the application but cited to understand the principle or theory underlying the invention |
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **12 April 2024** | **15 April 2024** |

| Name and mailing address of the ISA/KR | Authorized officer |
|---|---|
| **Korean Intellectual Property Office Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**

International application No.

**PCT/KR2023/021640**

**C.      DOCUMENTS CONSIDERED TO BE RELEVANT**

| Category* | Citation of document, with indication, where appropriate, of the relevant passages | Relevant to claim No. |
|---|---|---|
| A | KR 10-2008-0108687 A (SEOUL NATIONAL UNIVERSITY INDUSTRY FOUNDATION) 16 December 2008 (2008-12-16)<br>        See entire document. | 1-11 |

Form PCT/ISA/210 (second sheet) (July 2022)

## INTERNATIONAL SEARCH REPORT
### Information on patent family members

International application No.

**PCT/KR2023/021640**

| Patent document cited in search report | | | Publication date (day/month/year) | Patent family member(s) | | | Publication date (day/month/year) |
|---|---|---|---|---|---|---|---|
| JP | 2019-150648 | A | 12 September 2019 | AU | 2009-222165 | A1 | 11 September 2009 |
| | | | | AU | 2009-222165 | B2 | 09 July 2015 |
| | | | | AU | 2015-238865 | A1 | 29 October 2015 |
| | | | | AU | 2015-238865 | B2 | 17 November 2016 |
| | | | | CA | 2716896 | A1 | 11 September 2009 |
| | | | | CA | 2716896 | C | 09 August 2016 |
| | | | | CN | 102014975 | A | 13 April 2011 |
| | | | | CN | 105688276 | A | 22 June 2016 |
| | | | | EP | 2259805 | A2 | 15 December 2010 |
| | | | | EP | 2259805 | B1 | 17 August 2016 |
| | | | | EP | 3159018 | A2 | 26 April 2017 |
| | | | | EP | 3159018 | A3 | 10 May 2017 |
| | | | | EP | 3159018 | B1 | 20 April 2022 |
| | | | | ES | 2603961 | T3 | 02 March 2017 |
| | | | | JP | 2011-512957 | A | 28 April 2011 |
| | | | | JP | 2015-057126 | A | 26 March 2015 |
| | | | | JP | 2017-060889 | A | 30 March 2017 |
| | | | | JP | 6596200 | B2 | 23 October 2019 |
| | | | | US | 2011-0014258 | A1 | 20 January 2011 |
| | | | | US | 9011965 | B2 | 21 April 2015 |
| | | | | WO | 2009-111300 | A2 | 11 September 2009 |
| | | | | WO | 2009-111300 | A3 | 10 December 2009 |
| KR | 10-2009-0017693 | A | 18 February 2009 | AT | 477006 | T | 15 August 2010 |
| | | | | AT | 494915 | T | 15 January 2011 |
| | | | | AT | E494915 | T1 | 15 January 2011 |
| | | | | AU | 2007-258948 | A1 | 21 December 2007 |
| | | | | AU | 2007-258948 | B2 | 24 January 2013 |
| | | | | AU | 2008-206819 | A1 | 24 July 2008 |
| | | | | AU | 2008-206819 | B2 | 11 July 2013 |
| | | | | CA | 2654235 | A1 | 21 December 2007 |
| | | | | CA | 2654235 | C | 06 January 2015 |
| | | | | CA | 2670762 | A1 | 24 July 2008 |
| | | | | CA | 2670762 | C | 31 March 2015 |
| | | | | CN | 101466414 | A | 24 June 2009 |
| | | | | CN | 101466414 | B | 08 May 2013 |
| | | | | CN | 101578117 | A | 11 November 2009 |
| | | | | CN | 101578117 | B | 15 October 2014 |
| | | | | DK | 2026852 | T3 | 04 April 2011 |
| | | | | DK | 2101835 | T3 | 08 November 2010 |
| | | | | EP | 2026852 | A2 | 25 February 2009 |
| | | | | EP | 2026852 | B1 | 12 January 2011 |
| | | | | EP | 2101835 | A1 | 23 September 2009 |
| | | | | EP | 2101835 | B1 | 11 August 2010 |
| | | | | EP | 2316499 | A1 | 04 May 2011 |
| | | | | EP | 2316499 | B1 | 01 May 2013 |
| | | | | ES | 2358228 | T3 | 06 May 2011 |
| | | | | JP | 2009-539532 | A | 19 November 2009 |
| | | | | JP | 2010-515513 | A | 13 May 2010 |
| | | | | JP | 5268894 | B2 | 21 August 2013 |
| | | | | JP | 5535647 | B2 | 02 July 2014 |

Form PCT/ISA/210 (patent family annex) (July 2022)

**INTERNATIONAL SEARCH REPORT**
Information on patent family members

International application No.

**PCT/KR2023/021640**

| Patent document cited in search report | | Publication date (day/month/year) | Patent family member(s) | | | Publication date (day/month/year) |
|---|---|---|---|---|---|---|
| | | | KR | 10-2009-0099088 | A | 21 September 2009 |
| | | | PL | 2026852 | T3 | 30 March 2012 |
| | | | PL | 2101835 | T3 | 29 April 2011 |
| | | | US | 2009-0198344 | A1 | 06 August 2009 |
| | | | US | 2010-0136083 | A1 | 03 June 2010 |
| | | | WO | 2007-144667 | A2 | 21 December 2007 |
| | | | WO | 2007-144667 | A3 | 31 July 2008 |
| | | | WO | 2008-087448 | A1 | 24 July 2008 |
| US | 2018-0289858 A1 | 11 October 2018 | None | | | |
| KR | 10-2012-0075202 A | 06 July 2012 | KR | 10-1178537 | B1 | 30 August 2012 |
| KR | 10-2008-0108687 A | 16 December 2008 | KR | 10-0910064 | B1 | 30 July 2009 |

Form PCT/ISA/210 (patent family annex) (July 2022)